Europäisches Patentamt

European Patent Office

Office européen des brevets

(11) Veröffentlichungsnummer: **0 332 985 B1**

(19)

(12) **EUROPÄISCHE PATENTSCHRIFT**

(45) Veröffentlichungstag der Patentschrift: **11.11.92**

(51) Int. Cl.$^5$: **A61K 35/16**

(21) Anmeldenummer: **89103950.5**

(22) Anmeldetag: **07.03.89**

(54) Verfahren zum affinitätschromatografischen Reinigen von Faktor XIII.

(30) Priorität: **11.03.88 DE 3808048**

(43) Veröffentlichungstag der Anmeldung:
**20.09.89 Patentblatt 89/38**

(45) Bekanntmachung des Hinweises auf die
Patenterteilung:
**11.11.92 Patentblatt 92/46**

(84) Benannte Vertragsstaaten:
**AT BE CH DE ES FR GB GR IT LI LU NL SE**

(56) Entgegenhaltungen:
**DE-A- 3 734 923**
**FR-A- 2 119 009**

(73) Patentinhaber: **BEHRINGWERKE Aktiengesellschaft
Postfach 1140
W-3550 Marburg 1(DE)**

(72) Erfinder: **Löbermann, Hartmut, Dr.
Mooswaldstrasse 7b
W-7801 Schallstadt(DE)**
Erfinder: **Römisch, Jürgen, Dr.
Haselhecke 48
W-3550 Marburg(DE)**
Erfinder: **Stüber, Werner, Dr.
Cölber Weg 12
W-3551 Lahntal(DE)**

(74) Vertreter: **Fischer, Hans-Jürgen, Dr. et al
Hoechst AG Zentrale Patentabteilung Postfach 80 03 20
W-6230 Frankturt am Main 80(DE)**

EP 0 332 985 B1

## Beschreibung

Die Erfindung bezieht sich auf ein Verfahren zum affinitätschromatographischen Reinigen der a-Untereinheit von Faktor XIII, eine therapeutische Zubereitung und die Verwendung der therapeutischen Zubereitung.

Der Organismus verfügt über zwei im Gleichgewicht stehende Systeme, um sich sowohl vor Blutverlust als auch vor Thrombosen zu schützen: das Gerinnungssystem und das fibrinolytische System. Das Zusammenspiel zwischen beiden Systemen gewährleitstet, daß zunächst zur Blutstillung unlösliche Fibrinpolymere entstehen, die während der Wundheilung durch den lytischen Vorgang der Fibrinolyse wieder abgebaut werden.

Die Bildung von stabilen Thromben als Endstufe des Gerinnungsvorganges ist dabei das Ergebnis eines kaskadenförmig ablaufenden vernetzten Prozesses, in dessen Verlauf jeweils ein bereits aktiviertes Enzym durch Proteolyse das in der Kaskade folgende Enzym aktiviert und dabei die Antwort des Organismus auf die eingetretene Verletzung amplifiziert. Einer der letzten kritischen Schritte dieses Prozesses ist die Polymerisation von Fibrinmonomeren. Die Fibrinmonomere lagern sich nach ihrer Bildung zunächst unter der Wirkung elektrostatischer Kräfte längs parallel aneinander. In diesem zustand sind sie jedoch nur durch Wasserstoffbrücken verbunden und können durch Wasserstoffbrücken lösende Reagenzien, z.B. Harnstoff, wieder verflüssigt werden. Eine kovalente Verbindung zwischen den Fibrinmonomeren findet dann in Gegenwart von Calciumionen durch Bildung von Peptidbindungen zwischen der verschiedenen Monomeren statt. Das für diese Netzbildung verantwortliche Enzym ist der aktivierte Faktor XIII, der sog. Faktor XIII a.

Faktor XIII ist ein Proenzym des Blutgerinnungssystems und läßt sich sowohl im Plasma als auch in Plättchen nachweisen. Im Plasma tritt es als ein Komplex von a-und b-Untereinheiten auf, die jedoch nicht kovalent miteinander verbunden sind, während die in den Plättchen vorkommende Form nur aus a-Untereinheiten besteht. Die beiden molekularen Formen von Faktor XIII haben die gleiche enzymatische Funktion. Nach der Aktivierung durch Thrombin und Calcium katalysiert der nunmehr aktivierte Faktor XIII (Faktor XIIIa) die Bildung von peptidähnlichen Bindungen zwischen bestimmten Lysin-und Glutaminresten im Fibrin, wodurch die Fibrinmonomeren kovalent zu einem Netzwerk verbunden werden.

Ein genetisch determinierter Mangel von Faktor XIII oder eine durch Inhibitoren verminderte Aktivierung von Faktor XIII führt zu ernsten Blutgerinnungsstörungen. Aus diesem Grund sind verschiedene Verfahren für die Reinigung sowohl des Plasma- als auch des Plättchenfaktors XIII entwickelt worden. Diese zum Teile sehr aufwendigen Verfahren beruhen auf bekannten Proteinreinigungsschritten, beispielsweise fraktionierte Äthanolpräzipitation, Ammoniumsulfat- und Polyäthylenglykolfällungen, sowie Ionenaustauschchromatographie oder Gelfiltration. Die Autoren Jan McDonagh et al. (Biochimica et Biophysica Acta, 446 (1976), 345-357) beschreiben erstmals ein affinitätschromatographisches Verfahren zum Reinigen von Faktor XIII. Sie setzen dafür an Agarose gekoppelte Quecksilberbenzoate ein. Das Verfahren sieht die reversible Bindung der a-Untereinheit des Plasmafaktor XIII an die Quecksilberverbindung vor. Jan McDonagh et al. behaupten, daß die b-Untereinheit des Plasmafaktors, die selber keine freien Thiolgruppen enthält und daher nicht mit der Chromatographiematrix wechselwirken kann, solange durch nichtkovalente Kräfte an die a-Untereinheit gebunden bleibt, bis diese durch Spaltung mit Thrombin präaktiviert und durch anschließende Behandlung mit Calcium vollends aktiviert wird. Eine Präparation reiner a-Untereinheiten ist daher nach dem von den genannten Autoren beschriebenen Verfahren nur nach Behandlung mit Calcium und Thrombin möglich. Darüberhinaus sind die von ihnen verwendeten Quecksilberverbindungen hochtoxisch; das beschriebene Verfahren läßt sich daher nicht für die Herstellung von Humantherapeutika verwenden.

Aufgabe der vorliegenden Erfindung war es daher, ein verbessertes Verfahren zum Reinigen der a-Untereinheit von Faktor XIII zur Verfügung zu stellen, in dem die Verwendung toxischer Verbindungen vermieden werden kann.

Diese Aufgabe wird erfindungsgemäß in einem Verfahren der obengenannten Gattung dadurch gelöst, daß die a-Untereinheit von Faktor XIII reversibel an eine für Disulfidaustauschreaktionen geeignete Trägermatrix gebunden und durch Reaktion mit einem reduzierenden Mittel von der Trägermatrix entfernt wird.

Die reversible Bindung der a-Untereinheit von Faktor XIII an für Disulfidaustauschreaktionen geeignete Trägermatrizen hat den Vorteil, daß die Verwendung toxischer Chromatographiematerialien vermieden werden kann. Darüberhinaus können alle in der Faktor XIII-haltigen Lösung enthaltenen Proteine aufgrund einer niedrigeren Reaktivität gegenüber freien Mercaptogruppen oder infolge eines von Faktor XIII verschiedenen Verhaltens bei der anschließenden Elution mit einem die Disulfidbrükken reduzierenden Mittel von Faktor XIII getrennt werden. In dem erfindungsgemäßen Verfahren können weiterhin die b-Untereinheiten des Plasmafaktor XIII von a-Untereinheiten entfernt werden. Es wurde überraschenderweise gefunden, daß der Plasmafaktor XIII-Komplex im Gegensatz zu der von McDonagh vertretenen Auffassung durch Inkubation in Calcium- oder Magnesium-haltiger Lösung in Abwesenheit von Thrombin in b-Untereinheiten und biologisch aktive a-Untereinheiten zerfällt. Da die b-Untereinheiten selbst keine freien Mercaptogruppen aufweisen, sondern sämtliche durch Cysteine zur Verfügung gestellten SH-Gruppen als Disulfidbrücken innerhalb der b-Untereinheiten vorliegen, binden sie nicht an die Trägermatrix und können durch Waschen entfernt werden.

Faktor XIII wird bevorzugt aus Homogenaten von Materialien mit hoher Faktor XIII-Konzentration gewonnen. Dazu zählen beispielsweise Plazentagewebe und Blutplättchen. Der aus diesen beiden Materialien isolierte Faktor XIII besteht nur aus der a-Untereinheit.

Biologisch aktiver Faktor XIII kann ebenso aus Plasma gewonnen werden. Wie bereits erwähnt, besteht der Plasmafaktor XIII jedoch aus einem Komplex zweier nicht kovalent miteinander verbundener Untereinheiten, der a-un der b-Untereinheit. Erfindungsgemäß wird aus diesem Faktor XIII-Komplex die biologisch aktive a-Untereinheit gewonnen.

Für das erfindungsgemäße Verfahren wird Faktor XIII, gleich welcher Herkunft, für die affinitätschromatographische Reinigung in einer Lösung zur Verfügung gestellt. Dazu wird das entsprechende biologische Material z.B. durch Ausfällen oder Abzentrifugieren höhermolekularer Proteine aus dem Blut oder durch Homogenisieren und anschließendes Zentrifugieren von Faktor XIII-haltigem Gewebe vorbereitet.

Aus der Publikation von C.G. Curtis et al. (Biochemistry 13, 1974, 3774-3780) ist es bekannt, daß eine Behandlung von Faktor XIII mit alkylierenden Reagenzien in Abwesenheit von zweiwertigen Ionen der Gruppe IIa des Periodensystems keinen Einfluß auf die biologische Aktivität von Faktor XIII hat. Diese Autoren konnten zeigen, daß das aktive Cystein von Faktor XIIIa in der a-Untereinheit normalerweise geschützt vorliegt. Erst bei gleichzeitiger Zugabe von Calciumionen wird dieses Cystein durch Konformationsänderungen exponiert und daher ebenfalls einer Alkylierung zugänglich; der somit vollständig S-alkylierte Faktor XIIIa ist auch nach Spaltung durch Thrombin inaktiv. Überraschenderweise hat es sich gezeigt, daß durch Behandeln der Faktor XIII-haltigen Lösung mit alkylierenden Mitteln in Abwesenheit von Calcium oder gleichwertigen Ionen und nachfolgender affinitätschromatographischer Reinigung eine biologisch aktive Faktor XIII-Präparation höherer Reinheit erhalten werden kann.

Bevorzugt zu verwendende alkylierende Mittel sind dabei Jodacetamid oder Jodessigsäure. Erfindungsgemäß kann jedoch auch jedes andere Mittel gleicher Alkylierungskapazität verwendet werden.

Für die Alkylierung sollten die jeweiligen Alkylierungsmittel nach im Stand der Technik bekannten Vorschriften bei einer Konzentration von ungefähr 5 - 100 mmol/l verwendet werden. Eine bevorzugte Ausführungsform sieht die behandlung mit Jodessigsäure bei einer Konzentration von 5 - 50 mmol/l vor.

Erfindungsgemäß wird die Alkylierung in Abwesenheit von $Ca^{2+}$ oder gleichartig wirkenden Ionen durchgeführt. Ist dies nicht der Fall, so wird durch Konformationsänderungen der a-Untereinheit auch das aktive Zentrum einer Alkylierung zugänglich und damit blockiert. Sinnvollerweise werden daher $Ca^{2+}$ und $Mg^{2+}$-freie Puffer verwendet.

In einer bevorzugten Ausführungsform wird die Alkylierung in Anwesenheit von zugesetzten Chelatbildnern durchgeführt. Besonders bevorzugt ist dabei die Verwendung von Ethylendiamintetraessigsäure (EDTA) in einer Konzentration zwischen 0,001 - 0,1 mol/l.

Erfindungsgemäß wird die Alkylierung der Faktor XIII-haltigen Lösung bei einem pH durchgeführt, bei dem Faktor XIII keinen größeren Konformationsänderungen unterliegt. Bevorzugt wird daher ein pH in der Nähe des physiologischen pH-Wertes verwendet, d.h., die Alkylierung wird bei einem pH zwischen 6,5 und 8,5 durchgeführt. Um zu verhindern, daß durch $Ca^{2+}$-induzierte Änderungen der Tertiärstruktur des Moleküles das aktive Zentrum der a-Untereinheit freigelegt wird, werden dem Reaktionsansatz bevorzugt zwischen 0.002 und 0.02 mol/l EDTA zugesetzt. Die Alkylierung wird dann beispielsweise mit 0,005 - 0,25 mol/l Jodessigsäure durchgeführt. Es sind jedoch auch höhere oder niedrigere Konzentrationen der Jodessigsäure denkbar. Üblicherweise wird die Reaktion zwischen einer und 360 min durchgeführt. Um eine vollständige Alkylierung exponierter Mercaptogruppen zu erhalten, sollte die Reaktion jedoch länger als 5 min ablaufen. Der Reaktionszeit sind keine Grenzen gesetzt, jedoch kann nach spätestens 60 min mit einer ausreichenden Carboxy-Methylierung exponierter Mercaptogruppen gerechnet werden.

Nach Durchführen der Alkylierung wird die alkylierende Substanz aus der Lösung wieder entfernt. Dies kann durch dem Fachmann bekannte Verfahren, beispielsweise durch Gelfiltration, Salzfällung und/oder Dialyse, erfolgen. Jedes andere Verfahren ist dafür genauso anwendbar, vorausgesetzt, die alkylierende Substanz wird dadurch quantitativ aus der Lösung entfernt.

Bei der Isolierung der aktiven a-Untereinheit aus Plasmafaktor XIII muß diese von der nichtkovalent assoziierten b-Untereinheit abgetrennt werden. Dies geschieht bevorzugt durch eine Inkubation des gegebenenfalls bereits alkylierten Plasmafaktors XIII mit Calcium- oder Magnesiumionen in einer Konzentration von 0,05 - 0,6 mol/l, besonders bevorzugt 0,05 - 0,2 mol/l. Nach einer 5-minütigen biz zweistündigen Inkubation in Gegenwart von Calcium oder Magnesium liegen die a- und die b-Untereinheiten dissoziiert vor. Bei der Isolierung der a-Untereinheit aus Plasmafaktor XIII ist diese Dissoziation der Untereinheiten eine notwendige Voraussetzung für die Durchführung der nachfolgend beschriebenen Adsorption an für Disulfidaustauschreaktionen geeignetes Material.

Die entweder nicht vorbehandelte oder aber zuvor mit einer alkylierenden Substanz behandelte Faktor XIII-haltige Lösung wird anschließend mit der für Disulfidaustauschreaktion geeigneten Trägermatrix, die freie Mercaptogruppen enthält, in Kontakt gebracht. Ein bevorzugtes, für Disulfidaustauschreaktionen geeignetes Material ist z.B. Thiolsepharose 4B®. Die Reaktion kann sowohl im sogenannten Batch-Verfahren als auch auf präparativen Säulen stattfinden.

Erfindungsgemäß wird die Faktor XIII-haltige Lösung in Gegenwart von Calcium- oder Magnesiumionen, bevorzugt in einer Konzentration von 0,01 - 0,2 mol/l, mit der Matrix in Kontakt gebracht. Die Adsorption sollte bei nahezu physiologischem pH, bevorzugt bei einem pH zwischen 6,5 und 8,5, stattfinden.

Die Mercaptogruppen der Trägermatrix werden erfindungsgemäß zuvor in an sich bekannter Weise durch Umsetzen mit Di-2-pyridyldisulfid, $2,2'$-Dithiobispyridin-N-oxid, 2,2-Dinitro-5,5-dithiodibenzoesäure, Derivaten dieser Verbindungen oder Derivaten des Azodicarbonsäure aktiviert und somit für die Disulfidbrückenbindung mit der später applizierten Probe vorbereitet.

Nach der Bindung des Faktors XIII an die mercaptogruppenhaltige Matrix wird diese Matrix mit einer Lösung gewaschen, die Calcium oder Magnesium in einer Konzentration von 0,01 - 0,2 mol/l enthält. Alle weiteren Bestandteile der Faktor XIII-haltigen Lösung, die mit der Matrix nicht wechselwirken können, sei es, weil sie selber keine Cysteine enthalten, oder aber, weil ihre Mercaptogruppen zuvor alkyliert worden sind, werden quantitativ eluiert, im Falle des Faktor XIII aus Plasma ebenso die durch Calcium- oder Magnesiumbehandlung von der a-Untereinheit getrennten b-Untereinheiten.

In einem folgenden Verfahrensschritt wird die a-Untereinheit des Faktor XIII von der Matrix eluiert. Als Elutionsmittel kann dabei jedes für Disulfidaustauschreaktionen geeignete Mittel, das die an die Matrix gekoppelte Faktor XIII a-Untereinheit substituiert, und dabei die Disulfidbindung zwischen Matrix und a-Untereinheit reduziert, verwendet werden. Eine bevorzugte Ausführungsform sieht die Elution mit 0,005 - 0,05 mol/l eines reduzierenden Mittels, bevorzugt Dithiotreitol, Mercaptoethanol, Ethandithiol, Glutathion oder Cystein in Anwesenheit von 0,01 - 0,2 mol/l $Ca^{2+}$ oder $Mg^{2+}$ vor. Eine besonders bevorzugte Ausführungsform, die zu einer Erhöhung der Ausbeute aktiver a-Untereinheit führt, sieht den Zusatz von 0,1 - 0,7 g Saccharose/ml Elutionsmittel vor.

Dieses Verfahren kann nicht nur auf aus natürlichen Quellen isolierten Faktor XIII angewendet werden, sondern ebenso auf gentechnisch in Bakterien, Hefen oder Zellkulturen hergestellten Faktor XIII. Darüberhinaus ist es auf jedes Protein mit Faktor XIII-Aktivität anwendbar, vorausgesetzt, das Cystein des aktiven Zentrums unterliegt weiterhin den Einflüssen einer Konformationsänderung durch Zugabe von Calciumionen oder Ionen gleicher Wirkung.

Das erfindungsgemäße Verfahren führt zu einer Faktor XIII-Präparation, deren Aktivität während der Reinigung nahezu vollständig erhalten bleibt.

Der nach dem erfindungsgemäßen Verfahren gewonnene Faktor XIII kann für therapeutische Zubereitungen verwendet werden. Bevorzugte Zubereitungen sind dabei Faktor XIII-haltige Lösungen, die den Faktor XIII neben physiologisch verträglichen Bestandteilen, z.B. Natriumchlorid, Albumin, Glukose oder Saccharose enthalten, und für Infusionen geeignet sind.

Ein derartiges Therapeutikum kann vor allem zur Therapie von Faktor XIII-Mangelerkrankungen verwendet werden. Eine weitere bevorzute Verwendung des erfindungsgemäß gewonnen Faktor XIII ist z.B. die Zumischung zu Blutkonserven oder Erythrozytenkonzentraten, damit die Gerinnungsfähigkeit des nach großen Blutverlusten zugeführten Blutes erhalten bleibt.

Das erfindungsgemäße Verfahren zum Reinigen von Faktor XIII wird durch die folgenden Beispiele erläutert.

Beispiel 1

Aktivierung von Thiol-Sepharose 4B® mit Di-2-pyridyldisulfid, Ellmann's Reagenz oder $2,2'$-Dithiobis-pyridin-N-oxid

50 ml Thiol-Sepharose 4B® wurden zunächst 30 min bei Raumtemperatur (RT) mit Puffer A (0,05 mol/l Tris/HCl pH 7,5, 0,15 mol/l NaCl), dem 0,03 mol/l Mercaptoethanol zugefügt worden waren, behandelt. Anschließend wurde das Harzmaterial mit Puffer A gewaschen und mit diesem Puffer, dem zusätzlich 1,5 mmol/l Di-2-pyridyldisulfid zugefügt worden waren, umgesetzt. Nach vollständiger Aktivierung und Schutz der Mercaptogruppen der Harzmatrix wurde mit Puffer A gewaschen und die Harzmatrix für die Disulfidaustauschreaktion eingesetzt.

Anstelle von Di-2-pyridyldisulfid können beispielsweise in gleicher Molarität für die Aktivierung auch $2,2'$-Dinitro-5,5$'$-dithiodibenzoesäure (Ellmann's Reagenz) oder $2,2'$-Dithiobis-pyridin-N-oxid verwendet werden.

**Beispiel 2**

Umsetzung einer Faktor XIII-haltigen Lösung mit aktivierter Thiol-Sepharose 4B®

50 ml einer Faktor XIII-haltigen Lösung, die 700 E Faktor XIII (1 E Faktor XIII entspricht dem Gehalt an Faktor XIII in 1 ml Citrat-Normalplasma) enthielt, wurden mit 40% (w/v) Ammoniumsulfat gesättigt.

Der Ammoniumsulfatrückstand wurde über Nacht bei 4°C gegen Puffer A (Beispiel 1) dialysiert und anschließend mit 30 mmol/l CaCl$_2$ versetzt. Das aktivierte Thiolharz, das beispielsweise wie in Beispiel 1 beschrieben aktiviert worden war, wurde mit Puffer A, dem 30 mmol/l CaCl$_2$ zugesetzt worden waren, äquilibriert. Das Calcium-haltige Dialysat wurde auf die vorbereitete Affinitätsmatrix aufgegeben, das Harzmaterial anschließend mit Puffer A, 30 mmol/l CaCl$_2$ gewaschen und Faktor XIII mit Puffer A, dem 15 mmol/l Dithiothreitol und 30 mmol/l CaCl$_2$ zugesetzt worden waren, eluiert. Das Eluat enthielt 500 E Faktor XIII/

**Beispiel 3**

Die Aktivierung der Thiol-Sepharose 4B® und ihre Umsetzung mit einer Faktor XIII-haltigen Lösung wurden wie in den Beispielen 1 und 2 beschrieben durchgeführt, jedoch wurden dem Wasch- und Elutionspuffer zusätzlich 0,5 g Saccharose/ml Lösung zugesetzt. Das Eluat enthielt 600 E Faktor XIII.

**Beispiel 4**

Umsetzung einer Plasmafaktor XIII-haltigen Lösung mit aktivierten Thiol-Sepharose 4B®

50 ml einer Plasmafaktor XIII-haltigen Lösung, die 1000 Einheiten Faktor XIII jedoch kein Thrombin enthielt, wurde über Nacht bei 4° C gegen Puffer A (Beispiel 1) dialysiert und anschließend mit 0,3 mol/l CaCl$_2$ und 0,1 g Saccharose/ml versetzt. Nach einer Inkubationszeit von 60 min bei 4° C wurde die Lösung auf ein Thiolharz gegeben, das, beispielsweise wie in Beispiel 1 beschrieben, aktiviert und mit Puffer B, bestehend aus 0,05 mol/l Tris/HCl pH 7,5, 0,15 mol/l NaCl, 0,1 mol/l CaCl$_2$ und 0,1 g Saccharose/ml, äquilibriert worden war. Anschließend wurde das Harzmaterial mit Puffer B gewaschen und mit Puffer B, dem 20 mmol/l L-Cystein zugesetzt worden waren, eluiert.

Das Eluat enthielt 820 E Faktor XIII und war frei von b-Untereinheiten.

**Beispiel 5**

Alkylieren einer F XIII-haltigen Lösung und anschließendes Umsetzen mit aktivierten Thiol-Sepharose 4B®

50 ml einer Faktor XIII-haltigen Lösung, die 700 E Faktor XIII enthielt, wurden gegen Puffer A, dem 5 mmol/l EDTA zugesetzt worden waren, dialysiert und mit 10 mmol/l Jodessigsäure für 1 h bei RT inkubiert. Die Lösung wurde mit 40% (w/v) Ammoniumsulfat gesättigt und der Niederschlag, wie in Beispiel 2 beschrieben, weiterverarbeitet.

Ohne Saccharosezusatz wurden 450 E Faktor XIII im Eluat, mit Saccharosezusatz im Wasch- und Elutionspuffer 580 E Faktor XIII wiedergefunden. Nach der Alkylierung wurde der Faktor XIII in deutlich höherer Reinheit vom Affinitätsharz eluiert als ein nicht alkyliertes Faktor XIII-Material.

Die biologische Aktivität von Faktor XIII in einer Lösung wurde mit einem Fibrinquervernetzungstest der Behringwerke AG ermittelt. Die Proben wurden dazu in einer Verdünnungsreihe mit Diäthylbarbiturat-Acetat-Puffer, pH 7,6, 1:5, 1:10, 1:20, 1:40. verdünnt. Anschließend wurden je 50 ul Probenverdünnung mit 0,1 ml Ca-Thrombin-Kaolin-Lösung versetzt und 10 min bei 37°C inkubiert. Danach wurden sofort je 1 ml 5% Monochloressigsäurelösung zugefügt, weitere 2 min inkubiert und nach kräftigem Schütteln die Kaolintrübung der Lösung festgestellt.

**Patentansprüche**
**Patentansprüche für folgende Vertragsstaaten : AT, BE CH, DE, FR, GB, IT, LI, LU, NL, SE**

1. Verfahren zum affinitätschromatographischen Reinigen der a-Untereinheit von Faktor XIII, dadurch gekennzeichnet, daß die a-Untereinheit von Faktor XIII aus einer für das erfindungsgemäße Verfahren hergestellten Lösung von Faktor XIII unterschiedlicher Herkunft. reversibel an eine für Disulfidaustauschreaktionen geeignete Trägermatrix gebunden und durch Reaktion mit einem Disulfidbrücken reduzierenden Mittel von der Trägermatrix entfernt wird.

2. Verfahren nach Anspruch 1, dadurch gekennzeichnet, daß die a-Untereinheit von Faktor XIII aus Plazentahomogenat oder Plättchenhomogenat oder Plasma gewonnen wird.

3. Verfahren nach Anspruch 1, dadurch gekennzeichnet, daß die a-Untereinheit von Faktor XIII aus Plasma gewonnen wird.

4. Verfahren nach Anspruch 1, dadurch gekennzeichnet, daß die Lösung von Faktor XIII mit einem alkylierenden Mittel vorbehandelt wird.

**5.** Verfahren nach Anspruch 4, dadurch gekennzeichnet, daß das alkylierende Mittel Jodacetamid oder Jodessigsäure ist.

**6.** Verfahren nach Anspruch 4, dadurch gekennzeichnet, daß die Alkylierung in Abwesenheit von $Ca^{2+}$ und von $Mg^{2+}$ stattfindet.

**7.** Verfahren nach Anspruch 4, dadurch gekennzeichnet, daß die Alkylierung in Anwesenheit chelatbildender Substanzen, bevorzugt Ethylendiamintetraessigsäure (EDTA) in einer Konzentration von 0,001 - 0,1 mol/l, besonders bevorzugt von 0,002 - 0,02 mol/l, stattfindet.

**8.** Verfahren nach den Ansprüche 1 und 3, dadurch gekennzeichnet, daß die a-Untereinheit des Faktors zuvor aus dem Faktor XIII-Komplex aus Plasma durch Inkubieren mit $Ca^{2+}$ oder $Mg^{2+}$ in einer Konzentration von 0,05 - 0,6 mol/l, bevorzugt 0,05 - 0,2 mol/l freigesetzt wird.

**9.** Verfahren nach Anspruch 1, dadurch gekennzeichnet, daß die Faktor XIII-haltige Lösung mit einer mercaptogruppenhaltigen Trägermatrix, bevorzugt Thiolsepharose® 4B, in Kontakt gebracht wird.

**10.** Verfahren nach Anspruch 1, dadurch gekennzeichnet, daß die Bindung an die Trägermatrix in Gegenwart von $Ca^{2+}$ oder $Mg^{2+}$, bevorzugt in einer Konzentration von 0,01 - 0,2 mol/l, bevorzugt bei pH 6,5 - 8,5, stattfindet.

**11.** Verfahren nach Anspruch 1, dadurch gekennzeichnet, daß die Trägermatrix zuvor durch Umsetzen mit Di-2-pyridyldisulfid, 2,2′-Dithiobispyridin-N-oxid, 2,2′-Dinitro-5,5′-dithiobenzoesäure, Derivaten dieser Verbindungen oder Derivaten der Azodicarbonsäure aktiviert worden ist.

**12.** Verfahren nach Anspruch 1, dadurch gekennzeichnet, daß an die Trägermatrix gebundenes Material mit 0,01 - 0,2 mol/l $Ca^{2+}$ oder $Mg^{2+}$ und 0,005 - 0,05 mol/l eines reduzierenden Mittels, bevorzugt Dithiothreitol, Mercaptoethanol, Ethandithiol, Glutathion oder Cystein, und gegebenenfalls 0,1 - 0,7 g Saccharose/ml eluiert wird.

**13.** Verfahren nach Anspruch 1, dadurch gekennzeichnet, daß der Faktor XIII das natürliche Protein, ein gentechnisch hergestellter Faktor XIII bzw. dessen a-Untereinheit und/oder jedes natürlich oder künstlich erzeugte Protein mit Faktor XIII-Aktivität ist.

**14.** Nach dem Verfahren nach Anspruch 1 gereinigte a-Untereinheit von Faktor XIII als Arzneimittel.

**Patentansprüche für folgende Vertragsstaaten : ES, GR**

**1.** Verfahren zum affinitätschromatographischen Reinigen der a-Untereinheit von Faktor XIII, dadurch gekennziechnet, daß die a-Untereinheit von Faktor XIII aus einer für das erfindungsgemäße Verfahren hergestellten Lösung von Faktor XIII unterschiedlicher Herkunft reversibel an eine für Disulfidaustauschreaktionen geeignete Trägermatrix gebunden und durch Reaktion mit einem reduzierenden Mittel von der Trägermatrix entfernt wird.

**2.** Verfahren nach Anspruch 1, dadurch gekennzeichnet, daß die a-Untereinheit von Faktor XIII aus Plazentahomogenat oder Plättchenhomogenat gewonnen wird.

**3.** Verfahren nach Anspruch 1, dadurch gekennzeichnet, daß die a-Untereinheit von Faktor XIII aus Plasma gewonnen wird.

**4.** Verfahren nach Anspruch 1, dadurch gekennzeichnet, daß die Lösung von Faktor XIII mit einem alkylierenden Mittel vorbehandelt wird.

**5.** Verfahren nach Anspruch 4, dadurch gekennzeichnet, daß das alkyierende Mittel Jodacetamid oder Jodessigsäure ist.

**6.** Verfahren nach Anspruch 4, dadurch gekennzeichnet, daß die Alkylierung in Abwesenheit von $Ca^{2+}$ un von $Mg^{2+}$ stattfindet.

**7.** Verfahren nach Anspruch 4, dadurch gekennzeichnet, daß die Alkylierung in Anwesenheit chelatbildender Substanzen, bevorzugt Ethylendiamintetraessigsäure (EDTA) in einer Konzentration von 0,001 - 0,1 mol/l, besonders bevorzugt von 0,002 - 0,02 mol/l, stattfindet.

**8.** Verfahren nach den Ansprüche 1 und 3, dadurch gekennzeichnet, daß die a-Untereinheit des Faktors zuvor aus dem Faktor XIII-Komplex aus Plasma durch Inkubieren mit $Ca^{2+}$ oder $Mg^{2+}$ in einer Konzentration von 0,05 - 0,6 mol/l, bevorzugt 0,05 - 0,2 mol/l freigesetzt wird.

**9.** Verfahren nach Anspruch 1, dadurch gekennzeichnet, daß die Faktor XIII-haltige Lösung mit einer mercaptogruppenhaltigen Trägermatrix, bevorzugt Thiolsepharose® 4B, in Kontakt gebracht wird.

10. Verfahren nach Anspruch 1, dadurch gekennzeichnet, daß die Bindung an die Trägermatrix in Gegenwart von $Ca^{2+}$ oder $Mg^{2+}$, bevorzugt in einer Konzentration von 0,01 - 0,2 mol/l, bevorzugt bei pH 6,5 - 8,5, stattfindet.

11. Verfahren nach Anspruch 1, dadurch gekennzeichnet, daß die Trägermatrix zuvor durch Umsetzen mit Di-2-pyridyldisulfid, 2,2'-Dithiobispyridin-N-oxid, 2,2'-Dinitro-5,5'-dithiobenzoesäure, Derivaten dieser Verbindungen oder Derivaten der Azodicarbonsäure aktiviert worden ist.

12. Verfahren nach Anspruch 1, dadurch gekennzeichnet, daß an die Trägermatrix gebundenes Material mit 0,01 - 0,2 mol/l $Ca^{2+}$ oder $Mg^{2+}$ und 0,005 - 0,05 mol/l eines reduzierenden Mittels, bevorzugt Dithiothreitol, Mercaptoethanol, Ethandithiol, Glutathion oder Cystein, und gegebenenfalls 0,1 - 0,7 g Saccharose/ml eluiert wird.

13. Verfahren nach Anspruch 1, dadurch gekennzeichnet, daß der Faktor XIII das natürliche Protein, ein gentechnisch hergestellter Faktor XIII bzw. dessen a-Untereinheit und/oder jedes natürlich oder künstlich erzeugte Protein mit Faktor XIII-Aktivität ist.

**Claims**
**Claims for the following Contracting States :**
**AT, BE, CH, DE, FR, GB, IT, LI, LU, NL, SE**

1. A method for the purification of the a subunit of factor XIII by affinity chromatography, which comprises the a subunit of factor XIII from a solution, prepared for the method according to the invention, of factor XIII of different origin being reversibly bound to a carrier matrix suitable for disulfide exchange reactions and being removed from the carrier matrix by reaction with a disulfide bridge reducing agent.

2. The method as claimed in claim 1, wherein the a subunit of factor XIII is obtained from placental homogenate or platelet homogenate or plasma.

3. The method as claimed in claim 1, wherein the a subunit of factor XIII is obtained from plasma.

4. The method as claimed in claim 1, wherein the solution of factor XIII is pretreated with an alkylating agent.

5. The method as claimed in claim 4, wherein the alkylating agent is iodoacetamide or iodacetic acid.

6. The method as claimed in claim 4, wherein the alkylation takes placed in the absence of $CA^{2+}$ and of $Mg^{2+}$.

7. The method as claimed in claim 4, wherein the alkylation takes place in the presence of chelating substances, preferably ethylenediaminetetraacetic acid (EDTA) in a concentration of 0.001 - 0.1 mol/l, particularly preferably of 0.002 - 0.02 mol/l.

8. The method as claimed in claims 1 and 3, wherein the a subunit of the factor is previously liberated from the plasma factor XIII complex by incubation with $Ca^{2+}$ or $Mg^{2+}$ in a concentration of 0.05 - 0.6 mol/l, preferably 0.05 - 0.2 mol/l.

9. The method as claimed in claim 1, wherein the factor XIII-containing solution is brought into contact with a carrier matrix which contains mercapto groups, preferably thiol-Sepharose$^R$4B.

10. The method as claimed in claim 1, wherein the binding to the carrier matrix takes place in the presence of $Ca^{2+}$ or $Mg^{2+}$, preferably in a concentration of 0.01 - 0.2 mol/l, preferably at pH 6.5 - 8.5.

11. The method as claimed in claim 1, wherein the carrier matrix has previously been activated by reaction with di-2-pyridyl disulfide, 2,2'-dithiobis(pyridine N-oxide), 5,5'-dithiobis(2-nitrobenzoic acid), derivatives of these compounds or derivatives of asodicarboxylic acid.

12. The method as claimed in claim 1, wherein material bound to the carrier matrix is eluted with 0.01 - 0.2 mol/l $Ca^{2+}$ or $Mg^{2+}$ and 0.005 - 0.05 mol/l of a reducing agent, preferably dithiothreitol, mercaptoethanol, ethanedithiol, glutathione or cysteine and, where appropriate, 0.1 - 0.7 g of sucrose/ml.

13. The method as claimed in claim 1, wherein the factor XIII is the natural protein, a factor XIII which has been produced by genetic manipulation, or the a subunit thereof, and/or any naturally or artificially generated protein having factor XIII activity.

14. The a subunit of factor XIII purified by the method as claimed in claim 1 as a pharmaceu-

tical.

**Claims for the following Contracting States : ES, GR**

1. A method for the purification of the a subunit of factor XIII by affinity chromatography, which comprises the a subunit of factor XIII from a solution, prepared for the method according to the invention, of factor XIII of different origin being reversibly bound to a carrier matrix suitable for disulfide exchange reactions and being removed from the carrier matrix by reaction with a reducing agent.

2. The method as claimed in claim 1, wherein the a subunit of factor XIII is obtained from placental homogenate or platelet homogenate.

3. The method as claimed in claim 1, wherein the a subunit of factor XIII is obtained from plasma.

4. The method as claimed in claim 1, wherein the solution of factor XIII is pretreated with an alkylating agent.

5. The method as claimed in claim 4, wherein the alkylating agent is iodoacetamide or iodacetic acid.

6. The method as claimed in claim 4, wherein the alkylation takes placed in the absence of $CA^{2+}$ and of $Mg^{2+}$.

7. The method as claimed in claim 4, wherein the alkylation takes place in the presence of chelating substances, preferably ethylenediaminetetraacetic acid (EDTA) in a concentration of 0.001 - 0.1 mol/l, particularly preferably of 0.002 - 0.02 mol/l.

8. The method as claimed in claims 1 and 3, wherein the a subunit of the factor is previously liberated from the plasma factor XIII complex by incubation with $Ca^{2+}$ or $Mg^{2+}$ in a concentration of 0.05 - 0.6 mol/l, preferably 0.05 - 0.2 mol/l.

9. The method as claimed in claim 1, wherein the factor XIII-containing solution is brought into contact with a carrier matrix which contains mercapto groups, preferably thiol-Sepharose$^R$4B.

10. The method as claimed in claim 1, wherein the binding to the carrier matrix takes place in the presence of $Ca^{2+}$ or $Mg^{2+}$, preferably in a concentration of 0.01 - 0.2 mol/l, preferably at pH 6.5 - 8.5.

11. The method as claimed in claim 1, wherein the carrier matrix has previously been activated by reaction with di-2-pyridyl disulfide, 2,2'-dithiobis(pyridine N-oxide), 5,5'-dithiobis(2-nitrobenzoic acid), derivatives of these compounds or derivatives of azodicarboxylic acid.

12. The method as claimed in claim 1, wherein material bound to the carrier matrix is eluted with 0.01 - 0.2 mol/l $Ca^{2+}$ or $Mg^{2+}$ and 0.005 - 0.05 mol/l of a reducing agent, preferably dithiothreitol, mercaptoethanol, ethanedithiol, glutathione or cysteine and, where appropriate, 0.1 - 0.7 g of sucrose/ml.

13. The method as claimed in claim 1, wherein the factor XIII is the natural protein, a factor XIII which has been produced by genetic manipulation, or the a subunit thereof, and/or any naturally or artificially generated protein having factor XIII activity.

**Revendications**
**Revendications pour les Etats contractants suivants : AT, BE, CH, DE, FR, GB, IT, LI, LU, NL, SE**

1. Procédé de purification de la sous-unité a du facteur XIII, par chromatographie d'affinité, caractérisé en ce qu'on fixe réversiblement la sous-unité a du facteur XIII, provenant d'une solution de facteur XIII, d'origine diverse, préparée pour le procédé de l'invention, à une matrice support appropriée aux réactions d'échange de disulfure, et on la sépare de la matrice support par réaction avec un agent réducteur des ponts disulfure.

2. Procédé selon la revendication 1, caractérisé en ce que la sous-unité a du facteur XIII est obtenue à partir d'homogénéisat de placenta, d'homogénéisat de plaquettes, ou de plasma.

3. Procédé selon la revendication 1, caractérisé en ce que la sous-unité a du facteur XIII est obtenue à partir de plasma.

4. Procédé selon la revendication 1, caractérisé en ce que la solution de facteur XIII est prétraitée avec un agent alkylant.

5. Procédé selon la revendication 4, caractérisé en ce que l'agent alkylant est l'iodoacétamide ou l'acide iodoacétique.

6. Procédé selon la revendication 4, caractérisé en ce que l'alkylation a lieu en l'absence de $Ca^{2+}$ et de $Mg^{2+}$.

7. Procédé selon la revendication 4, caractérisé en ce que l'alkylation a lieu en présence de substances chélatantes, de préférence d'acide éthylènediaminetétracétique (EDTA) à une concentration de 0.001 à 0,1 mole/l, et tout particulièrement de 0,002 à 0,02 mole/l.

8. Procédé selon les revendications 1 et 3, caractérisé en ce que la sous-unité a du facteur XIII est préalablement libérée du complexe facteur XIII plasmatique, par incubation avec $Ca^{2+}$ ou $Mg^{2+}$ à une concentration de 0,05 à 0,6 mole/l, de préférence de 0,05 à 0,2 mole/l.

9. Procédé selon la revendication 1, caractérisé en ce que la solution contenant le facteur XIII est mise en contact avec une matrice support renfermant des groupes mercapto, de préférence avec du Thiolsepharose[(R)] 4B.

10. Procédé selon la revendication 1, caractérisé en ce que la fixation à la matrice support a lieu en présence de $Ca^{2+}$ ou $Mg^{2+}$, de préférence à une concentration de 0,01 à 0,2 mole/l, et de préférence à pH 6,5-8,5.

11. Procédé selon la revendication 1, caractérisé en ce que la matrice support a d'abord été activée par réaction avec du di-2-pyridyldisulfure, du N-oxyde de 2,2'-dithiobispyridine, de l'acide 2,2'-dinitro-5,5'-dithiobenzoïque, des dérivés de ces composés ou des dérivés de l'acide azodicarboxylique.

12. Procédé selon la revendication 1, caractérisé en ce que le matériau fixé a la matrice support est élué avec 0,01 à 0,2 mole/l de $Ca^{2+}$ ou $Mg^{2+}$ et 0,005 à 0,05 mole/l d'un réducteur, de préférence le dithiothréitol, le mercaptoéthanol, l'éthanedithiol, le glutathion ou la cystéine, et éventuellement avec 0,1 à 0,7 g de saccharose/ml.

13. Procédé selon la revendication 1, caractérisé en ce que le facteur XIII est la protéine naturelle, un facteur XIII ou sa sous-unité a produit par génie génétique, et/ou toute protéine naturelle ou synthétique présentant une activité de facteur XIII.

14. Sous-unité a du facteur XIII, purifiée suivant le procédé de la révendication 1, comme médicament.

**Revendications pour les Etats contractants suivants : ES, GR**

1. Procédé de purification de la sous-unité a du facteur XIII, par chromatographie d'affinité, caractérisé en ce qu'on fixe réversiblement la sous-unité a du facteur XIII, provenant d'une solution de facteur XIII, d'origine diverse, préparée pour le procédé de l'invention, à une matrice support appropriée aux réactions d'échange de disulfure, et on la sépare de la matrice support par réaction avec un agent réducteur.

2. Procédé selon la revendication 1, caractérisé en ce que la sous-unité a du facteur XIII est obtenue à partir d'homogénéisat de placenta ou d'homogénéisat de plaquettes.

3. Procédé selon la revendication 1, caractérisé en ce que la sous-unité a du facteur XIII est obtenue à partir de plasma.

4. Procédé selon la revendication 1, caractérisé en ce que la solution de facteur XIII est prétraitée avec un agent alkylant.

5. Procédé selon la revendication 4, caractérisé en ce que l'agent alkylant est l'iodoacétamide ou l'acide iodoacétique.

6. Procédé selon la revendication 4, caractérisé en ce que l'alkylation a lieu en l'absence de $Ca^{2+}$ et de $Mg^{2+}$.

7. Procédé selon la revendication 4, caractérisé en ce que l'alkylation a lieu en présence de substances chélatantes, de préférence d'acide éthylènediaminetétracétique (EDTA) à une concentration de 0,001 a 0,1 mole/l, et tout particulièrement de 0,002 à 0,02 mole/l.

8. Procédé selon les revendications 1 et 3, caractérisé en ce que la sous-unité a du facteur XIII est préalablement libérée du complexe facteur XIII plasmatique, par incubation avec $Ca^{2+}$ ou $Mg^{2+}$ à une concentration de 0,05 à 0,6 mole/l, de préférence de 0,05 à 0,2 mole/l.

9. Procédé selon la revendication 1, caractérisé en ce que la solution contenant le facteur XIII est mise en contact avec une matrice support renfermant des groupes mercapto, de préférence avec du Thiolsepharose[(R)] 4B.

10. Procédé selon la revendication 1, caractérisé en ce que la fixation à la matrice support a lieu en présence de $Ca^{2+}$ ou $Mg^{2+}$, de préférence

à une concentration de 0,01 à 0,2 mole/l, et de préférence à pH 6,5-8,5.

11. Procédé selon la revendication 1, caractérisé en ce que la matrice support a d'abord été activée par réaction avec du di-2-pyridyldisulfure, du N-oxyde de 2,2'-dithiobispyridine, de l'acide 2,2'-dinitro-5,5'-dithiodibenzoïque, des dérivés de ces composés ou des dérivés de l'acide azodicarboxylique.

12. Procédé selon la revendication 1, caractérisé en ce que le matériau fixé à la matrice support est élué avec 0,01 à 0,2 mole/l de $Ca^{2+}$ ou $Mg2^+$ et 0,005 à 0,05 mole/l d'un réducteur, de préférence le dithiothréitol, le mercaptoéthanol, l'éthanedithiol, le glutathion ou la cystéine, et éventuellement avec 0,1 à 0,7 g de saccharose/ml.

13. Procédé selon la revendication 1, caractérisé en ce que le facteur XIII est la protéine naturelle, un facteur XIII ou sa sous-unité a produit par génie génétique, et/ou toute protéine naturelle ou synthétique présentant une activité de facteur XIII.